(19) Europäisches Patentamt
European Patent Office
Office européen des brevets

(11) **EP 1 790 289 A2**

(12) **EUROPEAN PATENT APPLICATION**

(43) Date of publication:
**30.05.2007 Bulletin 2007/22**

(51) Int Cl.:
***A61B 6/00*** (2006.01)     ***A61B 6/03*** (2006.01)

(21) Application number: **06022627.1**

(22) Date of filing: **30.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **02.11.2005 JP 2005319913**

(71) Applicants:
• **KABUSHIKI KAISHA TOSHIBA**
  **Minato-ku,**
  **Tokyo 105-8001 (JP)**
• **Toshiba Medical Systems Corporation**
  **Otawara-shi, Tochigi-ken 324-8550 (JP)**

(72) Inventors:
• **Toyoshima, Naoko,**
  **c/o Intellectual Prop. Dept.,**
  **Otawara-shi**
  **Tochigi324-8550 (JP)**
• **Fujisawa, Yasuko,**
  **c/o Intellectual Prop. Dept.,**
  **Otawara-shi**
  **Tochigi324-8550 (JP)**

(74) Representative: **Kramer - Barske - Schmidtchen**
  **Radeckestrasse 43**
  **81245 München (DE)**

(54) **X-ray computed tomography apparatus and method of analyzing X-ray computed tomogram data**

(57) An X-ray computed tomography apparatus (1) calculates transfer functions h(t) using a time-concentration curve Ca(t) for pixels of a cerebral artery and a time-concentration curve Ci(t) for pixels of cerebral tissues including capillary vessels and then calculates a delay time for each pixel of an object region from a time when an artery curve rises on the basis of the transfer functions. Further, the apparatus (1) differently produces a visual color map showing time differences of blood flow conditions and provide it in a predetermined form by coloring pixels with a time delay from the other pixels without a time delay on the basis of the calculated delay time.

*FIG. 6*

Printed by Jouve, 75001 PARIS (FR)

EP 1 790 289 A2

**Description**

The present invention relates to an X-ray computed tomography apparatus (hereafter, called X-ray CT apparatus) that can employ an analysis method of bloodstream (hereafter, CT perfusion).

An X-ray CT apparatus acquires images (tomograms) by calculating (reconstruct) CT values, which are an X-ray absorbing rate of tissues of the internals relative to water, on the basis of the absorbed amount of X-rays in an object. In common X-ray CT diagnosis using an X-ray CT apparatus, information about a form can be acquired from single CT images. Further, in CT perfusion, it is possible to acquire dynamic condition information of bloodstream around a focus by dynamic scan using angiographic CT and provide the information as virtual information. In recent years, high-speed scan becomes possible by multislice CT and dynamic scan of angiographic CT can also be utilized in wide fields.

The CT perfusion, for example, is a method called CBP study for calculating indexes about dynamic condition of bloodstream in capillary vessels in cerebral tissues. The CBP study finds indexes of CBP, CBV, MTT, and Err etc. that quantitatively indicate dynamic conditions of "bloodstream passing through capillary vessels" or a map of the indexes. The index CBP etc. that quantitatively indicates dynamic conditions of bloodstream in capillary vessels in cerebral tissues is expected as useful information for finding information about elapsed time from starting of cerebral ischemia, discrimination of lesion of ischemic cerebral blood vessels, expansion of capillary vessels, and blood flow speed etc. (e.g. JP-A-2003-116843).

However, as for conventional X-ray CT apparatuses, in CT perfusion, it is impossible to detect information about delay of bloodstream (or contrast agents) reaching an object region (bloodstream delay information). Accordingly, with the use of the inspection result of the CT perfusion, it is possible to specify diseased parts due to decrease of blood flow, but it is impossible to specify diseased parts due to delay of bloodstream.

In order to overcome the above problems, it is an object of the invention to provide an X-ray computed tomography apparatus that easily specify diseased parts due to delay of bloodstream by detecting bloodstream delay information using CT perfusion and providing the information as visual information, and a method of analyzing X-ray computed tomogram data.

According to an aspect of the present invention, an X-ray computed tomography apparatus is provided, including a data acquiring unit that acquires a plurality of time-series image about an object region where contrast agents are injected, a delay time calculating unit that calculates a delay time of a concentration change curve of the object region with respect to a concentration change curve of an artery on the basis of the plurality of images, and a map producing unit that produces a map image indicating distribution of the delay time.

According to another aspect of the present invention, a method of analyzing X-ray computed tomography image data using an X-ray computed tomography apparatus that acquires a plurality of X-ray computed tomograms of time series about an object region where contrast agents are injected is provided, including measuring bloodstream information of the object region and an artery using the acquired continuous images, calculating a delay time of a concentration curve of the object region with respect to a concentration change curve of artery on the basis of the images, and producing a map image that displays the distribution of the delay time.

The invention can be more fully understood from the following detailed description when taken in conjunction with the accompanying drawings, in which:

FIG. 1 is a block diagram illustrating an X-ray CT apparatus 1 according to an embodiment of the invention;
FIGS. 2A and 2B show transfer functions when block-circulant type SVD method is used;
FIGS. 3A to 3C are exemplary embodiment of a map of blood flow acquired by bloodstream information measurement;
FIG. 4 is an exemplary embodiment of a delay map corresponding to the map of blood flow of FIG. 3A;
FIG. 5 shows a combined image of a delay map and computed tomographic angiography; and
FIG. 6 is a flowchart showing process order of an X-ray CT apparatus 1 of the invention employing CT perfusion.

Preferred embodiments of the invention are described in detail hereafter with reference to accompanying drawings. Components having the same functions and configurations herein are represented by the same reference numerals, but, if needed, repetitively described.

Preferred embodiments for an X-ray CT apparatus of the invention will be described with reference to the accompanying drawings. A variety of type of X-ray CT apparatuses are used, such as a rotate/rotate type in which an X-ray tube and X-ray detector integrally rotate around an object and a stationary/rotate type in which a number of ring-shaped detecting elements are arrayed and only an X-ray tube rotates around an object, but any type of X-ray CT apparatus is applicable to the invention. However, the rotate/rotate type that is commonly used in recent years is described herein by way of an example.

In order to reconstruct a slice of tomogram data, projection data of about 360°, i.e. one revolution around an object or 180° + view angle in half scan is required. Any type of reconstruction, however, is applicable to the invention.

The main current in a mechanism for converting an incident X-ray into a charge is an indirect conversion that converts an X-ray into light using a fluorescent material, such as a scintillator, and then into a charge using a photoelectric transducer, such as a photodiode and a direct conversion that uses photoconductivity, i.e. generation of an electron-hole pair in a semiconductor by an X-ray and its movement to an electrode. Any type of the above conversion is useful in an X-ray detecting element, but the indirect conversion is described herein by way of an example.

Further, in recent years, a multitube type X-ray computed tomography apparatus including a rotary frame with a plurality of pairs of an X-ray tube and an X-ray detector has been developed and the peripheral technologies have also been developed accordingly. The present invention is applicable to any type of existing X-ray CT apparatus of single tube and multitube types. However, a single tube type is described herein by way of an example.

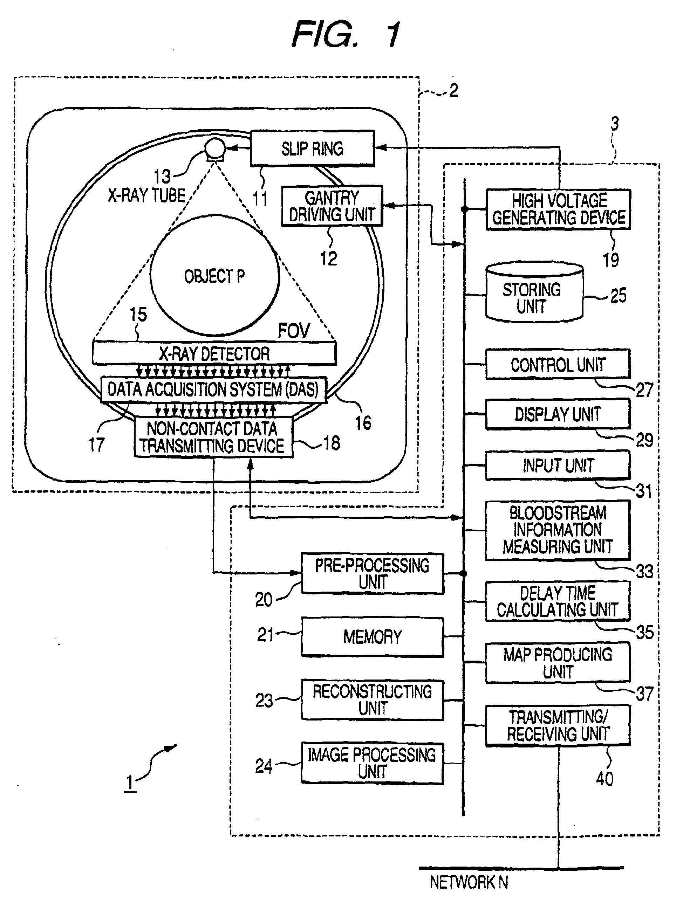

FIG. 1 is a block diagram for an X-ray CT apparatus 1 according to an embodiment of the invention. As shown in FIG. 1, an X-ray CT apparatus 1 is largely composed of a gantry 2, an information processing unit 3. Designed to acquire projection data about an object P, the gantry 2 has a slip ring 11, a gantry driving unit 12, an X-ray tube 13, an X-ray detector 15, a rotary frame 16, a data acquiring unit 17, and a not-contact data transmitting device 18. The information processing unit 3 controls the acquiring of data of the gantry 2 and processes the acquired data by the gantry 2 to create a X-ray CT image and various clinical information using it, and includes a high voltage generating device 19, a pre-processing unit 20, a memory unit 21, a reconstructing unit 23, an image processing unit 24, a storing unit 25, a control unit 27, a display unit 29, an input unit 31, a bloodstream information measuring unit 33, a delay time calculating unit 35, a map producing unit 37, and a transmitting/receiving unit 40.

The gantry driving unit 12 rotates the rotary frame 16. As the rotary frame 16 rotates, the X-ray tube 13 and the X-ray detector 15 facing each other spirally rotates about the body axis of an object P.

The X-ray tube 13 is a vacuum tube that generates X-rays, and mounted to the rotary frame 16. Power (tube current, tube voltage) required to radiate X-ray is provided from the high voltage generating device 19 to the X-ray tube 13 through the slip ring 11. The X-ray tube 13 radiates X-rays to an object within FOV (Field Of View) by accelerating and colliding charges with the target using the provided high voltage.

The X-ray detector 15 is a detector system that detects X-rays penetrating an object, and attached to the rotary frame 16 such that it faces the X-ray tube 13. The X-ray detector 15 is a single slice or a multislice type detector and a plurality of detecting elements formed by combining scintillators and photodiodes are arrayed in one dimension or two dimensions depending on each type.

The rotary frame 16 is a ring that rotates about the z-axis and includes the X-ray tube 13 and the X-ray detector 15 mounted thereon. The central region of the rotary frame 16 is open and an object P on a bed (not shown) is inserted through the opening.

The acquiring unit 17 is generally called DAS (data acquisition system) converts signals out of channels of the detector 15 into voltage signals and amplifies them, finally converts them into digital signals. The data (raw data) is transmitted into the information processing device 3 through the non-contact data transmitting device 18.

Providing power required to radiate X-rays to the X-ray tube 13 through the slip ring 11, the high voltage generating device 19 is composed of a high voltage transformer, a filament heating-converter, a rectifier, and a high voltage switch etc.

The pre-processing unit 20 receives raw data from the data acquiring system 17 through the non-contact data transmitting device 18 and then compensates the sensitivity and X-ray strength. The raw data for 360° undergoing a variety of compensation is temporally stored in the storing unit 25. The raw data undergoing the pre-process by pre-processing unit 20 is called as "projection data".

The reconstructing unit 23 employs a variety of reconstructing methods and reconstructs image data using a reconstructing method selected by an operator. The reconstructing methods include, for example, a fan beam reconstructing method (fan beam·convolution·back projection method), a FeldKamp method that is applied when projection rays cross a reconstructing surface with inclination and approximately reconstructing images by processing, on condition of small cone angle, considering a fan projection beam in folding or processing according to rays for scan in reverse projection, and a cone beam reconstructing method that prevents more cone angle errors compared with the FeldKamp method and compensates projection data depending on angles of rays against a reconstructing surface.

The image processing unit 24 applies image process for displaying, such as window conversion, RGB process etc., to the reconstructing image data produced by the reconstructing unit 23 and outputs the data through the display unit 29. Further, the image processing unit 24 produces pseudo three-dimensional images of tomograms for a cross section, projecting images in a direction, three-dimensional surface images, and outputs them through the display unit 29.

The storing unit 25 stores raw data, projection data, scanogram data, image data including tomogram data, and a program for diagnosis schedule etc. Further, the storing unit 25 stores a private program for measuring of bloodstream information, calculating of delay time, and drawing of a delay map that are described later.

The control unit 27 controls the X-ray CT apparatus 1 on the whole during scanning, signaling, image producing, image displaying etc. For example, the control unit 27, in the scanning, stores scanning conditions, such as slice thickness inputted in advance, in the memory 21 and controls sending amount and speed of the high voltage generating device 19, the bed driving unit 12, and a bed top plate a in their body axial direction, rotational speed and pitch of the X-ray tube 13 and the X-ray detector 15, and irradiating timing of the X-ray, and acquires (scans) X-ray CT image data by irradiating X-ray cone beams or X-ray fan beams to a desired imaging region of an object from a plurality of directions on the basis of scanning conditions automatically selected by a patient ID (for example, in a manual mode, scanning conditions that are directly set through an input unit 31).

The display unit 29 is an output device that displays CT images such as computed tomograms, scanograms, bloodstream information (CBP, CBV, MTT, and delay map (described later)) etc. inputted from the image processing unit 24. As for embodiments herein, the CT image may be defined as an image produced on the basis of each CT value within an imaging region acquired by an X-ray computed tomography apparatus. The CT value may be represented by an X-ray absorption coefficient of a material relative to a reference material (e.g. water). Further, the display unit 29 displays a scanning schedule that is constructed by a schedule supporting system (not shown).

The input unit 31 includes a keyboard, a variety of switches, and a mouse and allows an operator to input a variety of scanning conditions, such as the thickness and number of slices.

The bloodstream information measuring unit 33 measures bloodstream information represented by CBP study, which is described later in detail.

The delay time calculating unit 35 finds transfer functions from a curve of changes in time series for an artery (artery curve) and a curve of changes in time series for an object region (object curve) on the basis of bloodstream information produced by the bloodstream information measuring unit 33. Further the delay time calculating unit 35 calculates a delay time of the object curve from the transfer function for the artery curve. The calculating of the transfer function and the delay time are described later.

The map producing unit 37 produces a delay map that shows a difference (delay) between the artery and the object region on the time-axis on the basis of the calculated delay time. The producing of a delay map is also described later in detail.

The transmitting/receiving unit 40 communicates with other devices for image data and patient information etc. through a network N. In particular, the transmitting/receiving unit 40 receives information about imaging of an object (patient information and diagnostic region etc.) from an RIS (Radiology Information System) connected with the network N. (Bloodstream Information Measurement)

Bloodstream information measuring is described hereafter using CBP study by way of an example. In CBP study, indexes for CBP, CBV, MTT, and Err, which quantitatively represent dynamic characteristics of "the bloodstream passing through capillary vessels" in cerebral tissues, are acquired and a map for the index is outputted. Each index is defined as follows.

CBP: blood flow in capillary vessels of cerebral tissues per volume and time [ml/100 ml/min],
CBV: blood flow of cerebral tissues per volume [ml/100 ml]
MTT: average passing time of blood in capillary vessels [sec], and
Err: index of difference of actual measurements from analysis models. Analysis, such as discrimination of controlling tissues and controlled tissues of a cerebral artery, is possible according to the amount of the aforementioned index.

In the CBP study, contrast agents without cerebral blood vessel transmission are used as tracers, for example, an iodine contrast agent. An iodine agent is injected, for example, through a cubitus vein by an injector. An iodine agent that is intravenously injected by the injector flows a cerebral artery via a heart and lungs. The iodine agent flows into a cerebral vein from the cerebral artery through capillary vessels in cerebral tissues. Contrast agents without cerebral blood vessel transmission, for example, an iodine agent passes through capillary vessels without leaking out of the vessels in normal cerebral tissues.

The bloodstream information measuring unit 33 continuously acquires a passing condition of contrast agent as dynamic CTs and measures a time-concentration curve of pixels for a cerebral artery Ca(t), a time-concentration curve of pixels for a cerebral tissue including capillary vessel Ci(t), and a time-concentration curve of pixels for a cerebral vein Csss(t) from the continuous images. According to the CBP study, for blood concentration of the contrast agents, an ideal relationship between the curve Ca(t) of time-blood concentration for a cerebral vessels near cerebral tissues and the curve Ci(t) of time-blood concentration of capillary vessels is made as an analysis model, that is, when contrast agents are injected into a part of a blood vessel that is right before cerebral tissues, a time-concentration curve per a unit volume (1 pixel) of the cerebral tissues including capillary vessels vertically goes down with a slight slope. A transfer function may approximate to a rectangular function (bos-MTF method : box-Modulation Transfer Function method). Considering the time-blood concentration curve Ca(t) for a cerebral artery as an input function and the time-concentration curve Ci(t) for a cerebral tissue as an output function, properties in passing of capillary vessels may be found as a transfer function that is acquired by solving the following Formula 1.

(Calculating of Delay Time)

Calculating of delay time processed by the delay time calculating unit 35 is described hereafter. As for the present embodiment, it is assume that a relationship shown by the following Formula 1 is concluded among the curve Ca(t) for an artery acquired in the CBP study, each curve Ci(t) for object regions, and a transfer function h(t) between them.

$$C_i(t) = C_a(t) \otimes h(t) = \int_0^t C_a(t) \cdot h(t-\tau)d\tau \quad \ldots \text{(Formula 1)}$$

The inputted images are sampling into discrete times, so that when Formula 1 is discretized by a sampling time $\Delta t$, the following Formula 2 is obtained.

$$\begin{pmatrix} C_i(0) \\ C_i(1) \\ \vdots \\ C_i(N-1) \end{pmatrix} = \begin{pmatrix} C_a(0) & 0 & \cdots & 0 \\ C_a(1) & C_a(0) & 0 & \vdots \\ \vdots & & \ddots & 0 \\ C_a(N-1) & C_a(N-2) & \cdots & C_a(0) \end{pmatrix} \begin{pmatrix} h(0) \\ h(1) \\ \vdots \\ h(N-1) \end{pmatrix} \cdot \Delta t$$

$$\therefore Ci = Mc \cdot h \cdot \Delta t$$

$$\ldots \text{(Formula 2)}$$

Formula 2 shows simultaneous equations about the transfer functions h, so that the transfer functions h can be classified by solving the equations. However, because of clinical problems, the curve Ca(t) for an artery is not always contrasted before the curve Ci(t) for each object regions.

Assuming that the matrix for an artery Mc in Formula 2 is a block-circulant type, the following Formula 3 is obtained by modeling time-axial differences in position of the curves for an artery Ca(t) and each object regions Ci(t).

$$\begin{pmatrix} C_i(0) \\ C_i(1) \\ \vdots \\ C_i(N-1) \end{pmatrix} = \begin{pmatrix} C_a(0) & C_a(N-1) & \cdots & C_a(1) \\ C_a(1) & C_a(0) & C_a(N-1) & \vdots \\ \vdots & & \ddots & C_a(N-1) \\ C_a(N-1) & C_a(N-2) & \cdots & C_a(0) \end{pmatrix} \begin{pmatrix} h(0) \\ h(1) \\ \vdots \\ h(N-1) \end{pmatrix} \cdot \Delta t$$

$$\therefore Ci = Mb \cdot h \cdot \Delta t$$

$$\ldots \text{(Formula 3)}$$

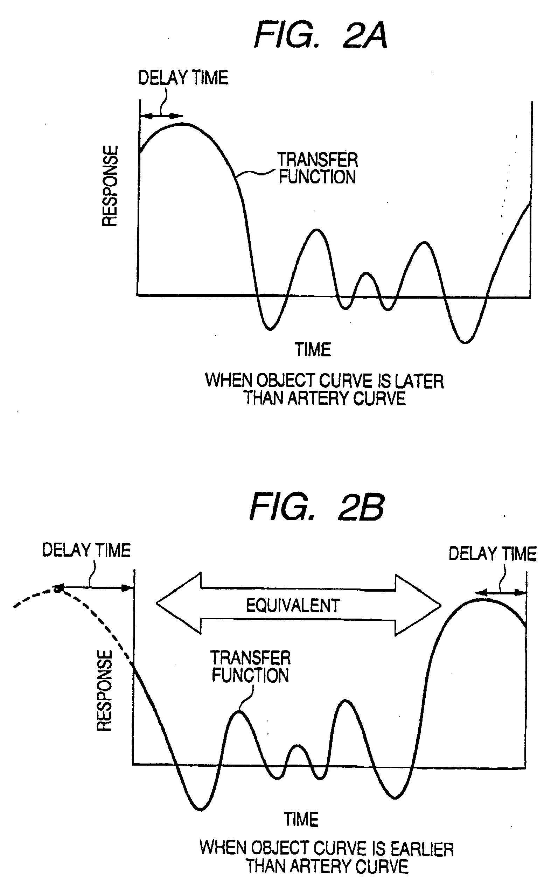

The transfer functions h(t) can be classified by solving the simultaneous equations of Formula 3. A variety of techniques are used for solving simultaneous equations, but when common SVD (Singular Value Decomposition) is used to solve simultaneous equations about a matter of fact, for example, the transfer functions shown in FIG. 2A (the object curve is later than the artery curve) and FIG. 2B (the object curve is earlier than the artery curve) are calculated. In the case shown in FIG. 2A, because the time for a peak of the transfer function is a time-axial difference between the artery curve Ca(t) and each object region curve Ci(t), the difference can be a delay time of each object pixel from the time when the artery curve rises.

A method of calculating the time for a peak of the transfer function h, i.e. the delay time is described hereafter. As for the transfer functions h(n) obtained by the block-circulant type SVD, the initial point (0, h(0)) and the final point (N-1, h(N-1)) are continuous and the transfer function is modeling to circulate. For this reason, as shown in FIG. 2B, when contrast agents reach the object region earlier than the artery, the negative time-axial peak of the transfer function returns to the final point (n=N-1). Accordingly, the data returned to the final point negative time-axially returns. Considering clinical problems, the largest possible difference in reaching-time of the contrast agent for the artery and object region is set to ten seconds and M pieces of data for ten seconds of the final point is negative time-axially moved by the following Formula 4.

$$M = 10.0/\Delta t$$

$$h(m) = h(N+m) \quad (m = -1, -2, \cdots -M) \quad \ldots \quad (Formula\ 4)$$

A time n=n_max for maximum value of the transfer function h(n) (n= -M, -M+1, ..., -1, 0, 1, ... N-M-1) is calculated, three points including two points before and after the above time (n_max-1, h(n_max-1)), (n_max, h(n_max)), (n_max+1, h(n_max+1) are interpolated into second order equation, and a time for the maximum value of the interpolated curve is a delay time.

As described above, the entire object image is analyzed using the same artery curve, so that it is possible to calculate a relative delay time for reaching of bloodstream of each pixel when the time when the contrast agent reaches the artery is 0. Further, a map can be used to display by producing images having delay times with respect to each pixel as pixel values.

Calculating of a delay time is described above according to an embodiment of the invention, but various modifications that are not limited to the above are possible. For example, according to the above embodiment, a method of calculating a delay time when the block-circulant type SVD method is used to classification of transfer functions. However, a method of classifying transfer functions is not limited to the block-circulant type SVD method. Further, even though the method of classifying transfer functions is not used, it is preferable to calculate a delay time from a difference between the times when the artery curve and the object region curve rise, respectively.

Further, in the above embodiment, when the contrast agents reach the object region earlier than the artery, the data turning to the final point is moved in the negative time-axis direction according to Formula 4. As for a position (pixel) where a negative delay time is obtained by the contrast agents that reach the object region earlier than the artery, the delay time (or pixel value) is 0 on condition that a delay map is not positively generated, or a predetermined color may be applied to show a negative delay time.

The image of a delay time is not limited to two dimensions, and when the input time series image is three dimensions, the image of a delay time may be three dimensions. In related cases, the measuring of bloodstream information and the calculating of delay time is applied to each voxel.

(Generating of a delay map with a delay time reflected)

Generating of a delay map with a delay time reflected is described hereafter. According to the generating of a delay map with a delay time reflected, as for pixels where a delay appears (e.g. pixels where a calculated delay time exceeds a predetermined critical value), a blood flow map with a color different from the pixel where a delay does not appear (e.g. a pixel where a calculated delay time does not exceed a predetermined critical value) is produced and a delay map is produced.

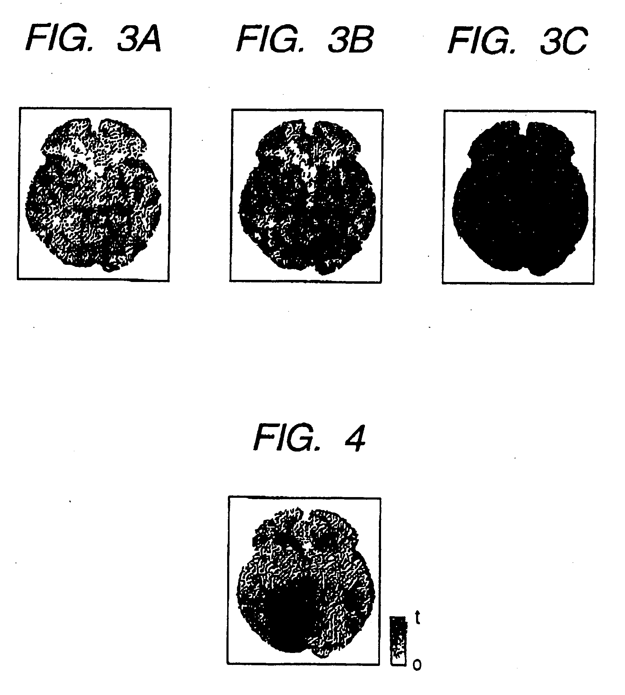

FIG. 3A shows an example of a blood flow map obtained by the measuring of bloodstream information. FIG. 4 shows an example of a delay map corresponding to the blood flow map of FIG. 3A. As seen by comparing FIG. 3A with 4, in the delay maps, different colors are applied to region where a delay time appears. Accordingly, an observer can easily see that blood is delayed at which region compared with the others by observing the delay maps. Further, because different colors are applied depending on delay times, an observer can also see the delay times by the colors.

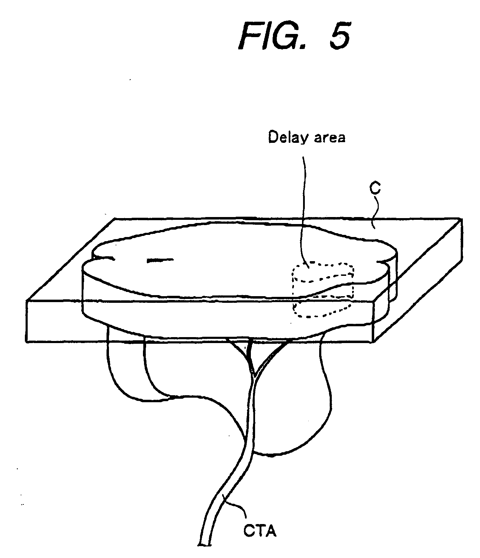

The delay maps may be displayed at the same time with the blood flow map or selectively displayed with the blood flow map. For example, as shown in FIG. 5, a combined image of a delay map C and CTA (Computed Tomographic Angiography) may be displayed. In particular, when a combined image is displayed, an observer can see the upstream artery of an object region that may be a reason for delay. In any display type, it is preferable to indicate to allow an observer to judge that which delay map is the information corresponding to which blood flow map.

(Operation)

The operation of the X-ray CT apparatus in CT perfusion is described hereafter. In the present embodiment, an example when CBP and a delay map corresponding to the CBP are produced considering a predetermined region inside a brain as an object region is described for more detailed description. However, not limited to the above, bloodstream information may be selected other than CBP, or the object region may be any region other than the inside of a brain.

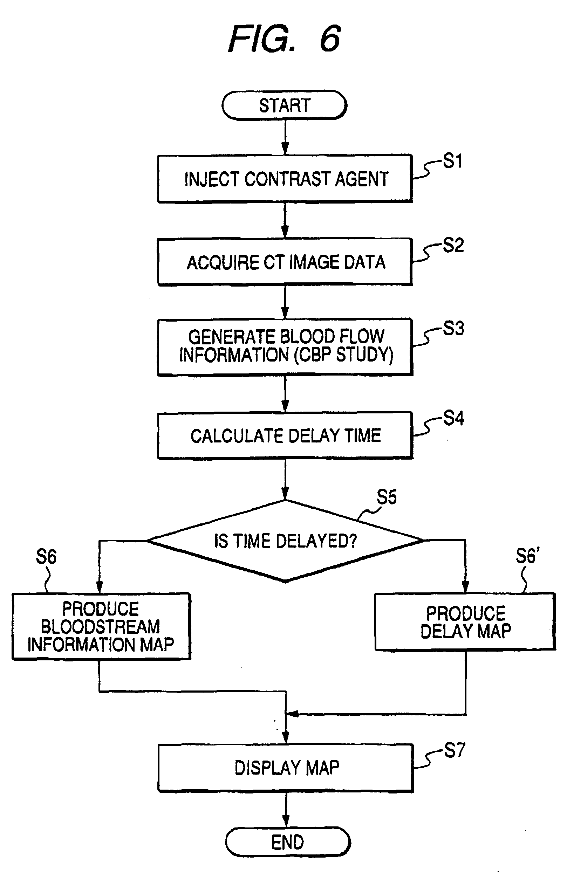

FIG. 6 shows a flowchart that illustrates an order of process by the X-ray CT apparatus 1 in CT perfusion. As seen from FIG. 6, contrast agents without cerebral blood vessel transmission, such as an iodine contrast agent, as tracers are intravenously injected into a cubitus vein, for example, by an injector (step S1). The iodine contrast agents intravenously injected by the injector flow into a cerebral artery via the heart and lungs. The contrast agents also flow into a cerebral vein from the cerebral artery via capillary vessels in the cerebral tissues. As for capillary vessels in normal cerebral tissues, the iodine contrast agents pass through (flow into) the vessels without leaking outside the vessels. On the contrary, the contrast agents leak outside the vessels in the capillary vessels in abnormal cerebral tissues. Further, in a vascular stricture region in the flowing passage, the iodine contrast agents slowly pass the vascular stricture region compared with a non-vascular stricture region in the flowing passage.

The control unit 27 collects data for imaging the passing conditions of the contrast agents injected in the object and generates continuous images through predetermined processes by applying dynamic CT (step S2). The bloodstream information measuring unit 33 measures at least a time-concentration curve $Ca(t)$ of pixels for cerebral artery and a time-concentration curve $Ci(t)$ of pixels for cerebral tissues including capillary vessels employing CBP study using the generated continuous images (step S3).

The delay time calculating unit 35 calculates transfer functions $h(t)$ using the measured time-concentration curves $Ca(t)$ of pixels for cerebral artery and the time-concentration curves $Ci(t)$ of pixels for cerebral tissues including capillary vessels, and calculates delay times for each pixel for an object region from the time when the artery curve rises on the basis of times for the peaks of the transfer functions $h(t)$ (step S4).

The control unit 27 determines whether the calculated delay times exceed a predetermined critical value (step S5), and when they do not exceed the predetermined critical value, the map producing unit 37 produces a common blood information map (CBP in this process) (step S6). On the contrary, when the control unit 27 determines that the delay times do not exceed a predetermined critical value, the map producing unit 37 applies colors corresponding to the delay times to the pixels where delay times appear and produces a delay map (step S6'). A critical value in step S5 is automatically set by an initial condition or manual operation, however, may be converted into an arbitrary value by a predetermined operation through the input unit 31.

The display unit 29 displays the produced bloodstream information map (CBP, delay map) in a predetermined form (step S7).

According to the above configuration, the following effects can be achieved.

According to the X-ray CT apparatus of the embodiment of the invention, transfer functions $h(t)$ are calculated using the time-concentration curves $Ca(t)$ about time of pixels for cerebral artery and the time-concentration curves $Ci(t)$ about time of pixels for cerebral tissues including capillary vessels, and delay times for each pixel for object regions from the time when the artery curve rises is calculated on the basis of the transfer functions $h(t)$. Further, the pixels where delay times appear are differently colored from the pixels where delay times do not appear on the basis of the calculated delay time; therefore, the time difference of blood flowing condition makes a visual color map and provides a predetermined form. Accordingly, an observer, such as a doctor etc., observes the provided color map and can easily specify the object regions and diseased parts by delay of bloodstream around the object regions.

Further, according to the X-ray CT apparatus of the embodiments of the invention, each pixel is colored depending on the calculated delay times. Accordingly, an observer, such as a doctor etc., is provided with a delay map by which the delay time can be recognized by intuition.

Furthermore, according to the X-ray CT apparatus of the embodiments of the invention, the transfer functions $h(t)$ are classified using the time-concentration curves $Ca(t)$ of pixels for a cerebral artery and the time-concentration curve $Ci(t)$ of pixels for cerebral tissues including capillary vessels, for example, using block-circulant type SVD method. According to the methods, delay times for each pixel for object region from the time when the artery curve rises can be relatively easily calculated on the basis of the time for the peaks of the transfer functions $h(t)$.

The present invention is not limited to the above embodiments and the components may be modified for each step without departing from aspects of the invention. Further, the present invention may be also modified in a variety of type by appropriately combining the above components. For example, some components of the whole components may be removed in the above embodiments.

The components may be appropriately combined in other embodiments.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

**Claims**

1.    An X-ray computed tomography apparatus (1) **characterized by** comprising:

a data acquiring unit (2) that acquires a plurality of time-series images of an object region to which contrast agents are injected;

a delay time calculating unit (35) that calculates a delay time of a concentration change curve of the object region with respect to a concentration change curve of an artery on the basis of the plurality of images; and

a map producing unit (37) that produces a map image indicating distribution of the delay time.

2. The X-ray computed tomography apparatus (1) according to claim 1,
   **characterized in that** the delay time calculating unit (35) calculates transfer functions of time-concentration curve for the object region with respect to a time-concentration curve for the artery using a block-circulant type SVD method, and calculates a delay time on the basis of peak time of the transfer functions.

3. The X-ray computed tomography apparatus (1) according to claim 1,
   **characterized in that** the delay time calculating unit (35) calculates a delay time on the basis of differences between a time when the concentration change curve of artery increases and a time when the concentration change curve of object region increases.

4. The X-ray computed tomography apparatus (1) according to claim 1,
   **characterized in that** the map producing unit (37) generates a color map that shows time differences of blood flow conditions by coloring depending on the delay time.

5. The X-ray computed tomography apparatus (1) according to claim 1,
   **characterized in that** the map producing unit (37) moves the concentration change curve of object region in the negative direction of the time-axis when the contrast agents reach the object region earlier than the artery, and produces a two-dimensional map image indicating distribution of a delay time on the basis of the concentration change curve of object region after the moving.

6. The X-ray computed tomography apparatus (1) according to claim 1,
   **characterized in that** the delay time is replaced the calculated value to other value when the contrast agents reach the object region earlier than the artery.

7. The X-ray computed tomography apparatus (1) according to claim 1, **characterized by** further comprising:

   a display unit (29) that simultaneously or selectively displays the two-dimensional map image and a blood flow map at the same time.

8. The X-ray computed tomography apparatus (1) according to claim 1, **characterized by** further comprising:

   a display unit (29) that displays a combined image of the two-dimensional map image and a computed tomographic angiography.

9. A method of analyzing X-ray computed tomography image data using an X-ray computed tomographic apparatus that acquires a plurality of time series X-ray computed tomograms of an object region to which contrast agents are injected, **characterized by** comprising:

   measuring bloodstream information of the object region and an artery using acquired continuous images;
   calculating a delay time of a concentration curve of the object region with respect to a concentration change curve of artery; and
   producing a map image that displays distribution of the delay time.

10. An Image processing apparatus **characterized by** comprising:

    a delay time calculating unit (35) that calculates a delay time of a concentration change curve of the object region with respect to a concentration change curve of an artery on the basis of the plurality of images; and
    a map producing unit (37) that produces a map image indicating distribution of the delay time.

# FIG. 1

# FIG. 2A

DELAY TIME

RESPONSE

TRANSFER
FUNCTION

TIME

WHEN OBJECT CURVE IS LATER
THAN ARTERY CURVE

# FIG. 2B

DELAY TIME

DELAY TIME

EQUIVALENT

RESPONSE

TRANSFER
FUNCTION

TIME

WHEN OBJECT CURVE IS EARLIER
THAN ARTERY CURVE

*FIG. 3A*    *FIG. 3B*    *FIG. 3C*

*FIG. 4*

# FIG. 5

Delay area

C

CTA

## FIG. 6

```
                    ┌──────────┐
                    │  START   │
                    └──────────┘
                          │
                          ▼
          ┌──────────────────────────────┐
          │     INJECT CONTRAST AGENT     │──S1
          └──────────────────────────────┘
                          │
                          ▼
          ┌──────────────────────────────┐
          │     ACQUIRE CT IMAGE DATA     │──S2
          └──────────────────────────────┘
                          │
                          ▼
          ┌──────────────────────────────┐
          │    GENERATE BLOOD FLOW        │──S3
          │    INFORMATION (CBP STUDY)    │
          └──────────────────────────────┘
                          │
                          ▼
          ┌──────────────────────────────┐
          │     CALCULATE DELAY TIME      │──S4
          └──────────────────────────────┘
                          │
                          ▼
                   ◇──────────────◇  S5
          ┌────────┤ IS TIME DELAYED? ├────────┐
          │        ◇──────────────◇            │
    S6    ▼                                     ▼   S6'
  ┌─────────────────┐                ┌─────────────────┐
  │    PRODUCE      │                │    PRODUCE      │
  │  BLOODSTREAM    │                │   DELAY MAP     │
  │ INFORMATION MAP │                └─────────────────┘
  └─────────────────┘                         │
          │                                    │
          └────────────────┬───────────────────┘
                           ▼
          ┌──────────────────────────────┐
          │        DISPLAY MAP            │──S7
          └──────────────────────────────┘
                          │
                          ▼
                    ┌──────────┐
                    │   END    │
                    └──────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003116843 A